# EUROPEAN PATENT APPLICATION

(11) **EP 3 578 257 A1**
(43) Date of publication of application: **11.12.2019**
(21) Application number: 17895068.9
(22) Date of filing: 18.12.2017
(51) Int. Cl.: B01J 23/83, B01J 23/78, B01J 37/04, B01J 37/08, C07C 1/02, C07C 9/04, C07B 61/00

(54) **METHANATION REACTION CATALYST, METHOD FOR PRODUCING METHANATION REACTION CATALYST, AND METHOD FOR PRODUCING METHANE**

(30) Priority: 01.02.2017 JP 2017016497
(71) Applicant: Hitachi Zosen Corporation, Osaka-shi, Osaka 559-8559 (JP)
(72) Inventor: MORI, Takuma, Osaka-shi Osaka 559-8559 (JP); NISHIDA, Yusuke, Osaka-shi Osaka 559-8559 (JP); SHONO, Emi, Osaka-shi Osaka 559-8559 (JP)
(74) Representative: Grünecker Patent- und Rechtsanwälte PartG mbB
(86) International application number: PCT/JP2017/045372
(87) International publication number: WO 2018/142787

(57) **Abstract**

A methanation reaction catalyst is for subjecting CO and/or CO₂ to methanation reaction with hydrogen, and is a calcined product of a wet mixture of zirconia and/or a salt of Zr, a salt of a stabilizing element, a salt of Ni, and an inorganic oxide. The stabilizing element is at least one of element selected from the group consisting of Y, La, Ce, Pr, Nd, Sm, Gd, Dy, Ca, Mg, Mn, Fe, and Co, and the inorganic oxide is at least one of inorganic oxide selected from the group consisting of silica, alumina, titania, and ceria. When the inorganic oxide contains the silica, the mole ratio of the Ni atom with respect to the Si atom is 0.20 or more and 10.0 or less. When the inorganic oxide contains the alumina, the mole ratio of the Ni atom with respect to the Al atom is 0.20 or more and 7.0 or less. When the inorganic oxide contains the titania, the mole ratio of the Ni atom with respect to the Ti atom is 0.30 or more and 10.5 or less. When the inorganic oxide contains the ceria, the mole ratio of the Ni atom with respect to the Ce atom is 0.70 or more and 25.0 or less.

## Description

### TECHNICAL FIELD

The present invention relates to a methanation reaction catalyst, a method for producing a methanation reaction catalyst, and a method for producing methane.

### BACKGROUND ART

A methanation reaction catalyst for subjecting CO and CO₂ to methanation reaction with hydrogen has been known. As such a methanation reaction catalyst, for example, a catalyst produced by reducing a catalyst precursor having the composition of Ni: 60.2 atom%, Zr: 20.6 atom%, Sm: 14.0 atom%, and Si: 5.1 atom% has been proposed (ref: for example, Patent Document 1).

The catalyst is produced as follows: a samarium nitrate hexahydrate, a nickel nitrate hexahydrate, and a silica sol are added to a hydrosol of zirconia to be kneaded, so that a pellet (kneaded product) to be obtained is calcined to obtain a catalyst precursor, and thereafter, the catalyst precursor is reduced.

### Citation List

### Patent Document

Patent Document 1: Japanese Unexamined Patent Publication No. 2013-119526

### SUMMARY OF THE INVENTION

### PROBLEM TO BE SOLVED BY THE INVENTION

However, in the catalyst described in Patent Document 1, the catalytic activity is insufficient, and there is a limit in achieving the improvement of the production rate of methane.

An object of the present invention is to provide a methanation reaction catalyst that is capable of achieving the improvement of the catalytic activity and the improvement of the methane production rate, a method for producing a methanation reaction catalyst, and a method for producing methane.

### MEANS FOR SOLVING THE PROBLEM

The present invention [1] includes a methanation reaction catalyst for subjecting CO and/or CO₂ to methanation reaction with hydrogen, and being a calcined product of a wet mixture of zirconia and/or a salt of Zr, a salt of a stabilizing element, a salt of Ni, and an inorganic oxide, wherein the stabilizing element is at least one of element selected from the group consisting of Y, La, Ce, Pr, Nd, Sm, Gd, Dy, Ca, Mg, Mn, Fe, and Co, and the inorganic oxide is at least one of inorganic oxide selected from the group consisting of silica, alumina, titania, and ceria; when the inorganic oxide contains the silica, the mole ratio of the Ni atom with respect to the Si atom is 0.20 or more and 10.0 or less; when the inorganic oxide contains the alumina, the mole ratio of the Ni atom with respect to the Al atom is 0.20 or more and 7.0 or less; when the inorganic oxide contains the titania, the mole ratio of the Ni atom with respect to the Ti atom is 0.30 or more and 10.5 or less; and when the inorganic oxide contains the ceria, the mole ratio of the Ni atom with respect to the Ce atom is 0.70 or more and 25.0 or less.

According to the structure, the methanation reaction catalyst is the calcined product of the wet mixture of zirconia and/or the salt of Zr, the salt of the stabilizing element, the salt of Ni, and the inorganic oxide, and the mole ratio of the Ni atom with respect to at least any one of the Si atom, the Al atom, the Ti atom, and the Ce atom is within the above-described range.

Thus, the inorganic oxide acts so as to form a space between the plurality of Ni atoms that are a catalytically active component, and suppresses the aggregation of Ni. As a result, the improvement of the fine pore volume in the methanation reaction catalyst can be achieved, and the improvement of the dispersibility of Ni can be achieved.

In this manner, the improvement of the specific surface area of the methanation reaction catalyst can be achieved, and an increase in the surface-exposed amount of Ni can be achieved. As a result, the improvement of the catalytic activity of the methanation reaction catalyst can be achieved, and the improvement of the methane production rate can be achieved.

The present invention [2] includes the methanation reaction catalyst described in the above-described [1], wherein when the inorganic oxide contains the silica, the mole ratio of the Zr atom with respect to the Si atom is 0.10 or more and 4.5 or less; when the inorganic oxide contains the alumina, the mole ratio of the Zr atom with respect to the Al atom is 0.10 or more and 4.0 or less; when the inorganic oxide contains the titania, the mole ratio of the Zr atom with respect to the Ti atom is 0.15 or more and 6.0 or less; and when the inorganic oxide contains the ceria, the mole ratio of the Zr atom with respect to the Ce atom is 0.30 or more and 12.0 or less.

According to the structure, the mole ratio of the Zr atom with respect to at least any one of the Si atom, the Al atom, the Ti atom, and the Ce atom is within the above-described range, so that the inorganic oxide acts so as to form a space between the plurality of particles of a zirconia support mainly composed of Zr, and the improvement of the fine pore volume in the methanation reaction catalyst can be further achieved.

In this manner, the improvement of the specific surface area of the methanation reaction catalyst can be further achieved, and the improvement of the methane production rate can be further achieved.

The present invention [3] includes the methanation reaction catalyst described in the above-described [1] or [2], wherein the content ratio of the inorganic oxide with respect to the total sum of the calcined product of the wet mixture of zirconia and/or the salt of Zr, the salt of the stabilizing element, the salt of Ni, and the inorganic oxide is 5 mass% or more and 62 mass% or less.

According to the structure, the content ratio of the inorganic oxide is within the above-described range, so that the improvement of the dispersibility of Ni can be stably achieved, and the improvement of the fine pore volume in the methanation reaction catalyst can be stably achieved.

The present invention [4] includes the methanation reaction catalyst described in any one of the above-described [1] to [3] having a plurality of fine pores, wherein the total sum of the volume of the fine pore having a fine pore diameter of 2 nm to 12 nm of the plurality of fine pores is 0.050 cm³/g or more per unit mass.

According to the structure, the total sum of the volume of the fine pore having a fine pore diameter of 2 nm to 12 nm is 0.050 cm³/g or more per unit mass, so that an increase in the surface-exposed amount of Ni in the methanation reaction catalyst can be further achieved, and the improvement of the catalytic activity of the methanation reaction catalyst can be further achieved.

The present invention [5] includes a method for producing methane bringing the methanation reaction catalyst described in any one of the above-described [1] to [4] into contact with a mixed gas containing CO and/or Co₂ and a hydrogen gas at 200°C or more.

According to the method, the catalytic activity of the methanation reaction catalyst is improved, so that the methane can be efficiently produced.

The present invention [6] includes a method for producing a methanation reaction catalyst including the steps of wet-mixing zirconia and/or a salt of Zr, at least one of inorganic oxide selected from the group consisting of silica, alumina, titania, and ceria, a salt of at least one of stabilizing element selected from the group consisting of Y, La, Ce, Pr, Nd, Sm, Gd, Dy, Ca, Mg, Mn, Fe, and Co, and a salt of Ni to prepare a mixture and calcining the mixture at 400°C or more and 800°C or less, wherein in the step of preparing the mixture, when the inorganic oxide contains the silica, the mixture is prepared so as to have the mole ratio of the Ni atom with respect to the Si atom of 0.20 or more and 10.0 or less; when the inorganic oxide contains the alumina, the mixture is prepared so as to have the mole ratio of the Ni atom with respect to the Al atom of 0.20 or more and 7.0 or less; when the inorganic oxide contains the titania, the mixture is prepared so as to have the mole ratio of the Ni atom with respect to the Ti atom of 0.30 or more and 10.5 or less; and when the inorganic oxide contains the ceria, the mixture is prepared so as to have the mole ratio of the Ni atom with respect to the Ce atom of 0.70 or more and 25.0 or less.

According to the method, zirconia and/or the salt of Zr, the inorganic oxide, the salt of stabilizing element, and the salt of Ni are wet-mixed so as to have the mole ratio of the Ni atom with respect to at least any one of the Si atom, the Al atom, the Ti atom, and the Ce atom within the above-described range to prepare the mixture, and thereafter, the mixture is calcined at 400°C or more and 800°C or less, so that the methanation reaction catalyst is produced.

Thus, even by an easy method, the improvement of the catalytic activity can be achieved, and the methanation reaction catalyst that is capable of achieving the improvement of the methane production rate can be produced.

### EFFECT OF THE INVENTION

According to the methanation reaction catalyst of the present invention, the improvement of the catalytic activity can be achieved, and the improvement of the methane production rate can be achieved.

According to the method for producing methane of the present invention, the methane can be efficiently produced.

According to the method for producing a methanation reaction catalyst of the present invention, the methanation reaction catalyst that is capable of achieving the improvement of the catalytic activity, and the improvement of the methane production rate can be produced.

### BRIEF DESCRIPTION OF THE DRAWINGS

FIG. 1 shows a graph illustrating the fine pore distribution in methanation reaction catalysts (catalysts before reduction) of Examples 4 to 6 and Comparative Examples 1 and 4.
FIG. 2 shows a graph illustrating the correlation between the mole ratio (Ni/Si) of the Ni atom with respect to the Si atom and the ratio (k/k₀) of the methane production rate of each of the methanation reaction catalysts with respect to the methane production rate of the methanation reaction catalyst of the additive-free inorganic oxide in the methanation reaction catalysts (catalysts after reduction) of Examples 1 to 14 and Comparative Examples 1 to 3 and 6 to 8.
FIG. 3 shows a graph illustrating the correlation between the mole ratio (Zr/Si) of the Zr atom with respect to the Si atom and the ratio (k/k₀) of the methane production rate in the methanation reaction catalysts (catalysts after reduction) of Examples 1 to 14 and Comparative Examples 1 to 3 and 6 to 8.
FIG. 4 shows a graph illustrating the correlation between the mole ratio (Ni/Al) of the Ni atom with respect to the Al atom and the ratio (k/k₀) of the methane production rate in the methanation reaction catalysts (catalysts after reduction) of Examples 15 to 24 and Comparative Examples 9 to 12.
FIG. 5 shows a graph illustrating the correlation between the mole ratio (Zr/Al) of the Zr atom with respect to the Al atom and the ratio (k/k₀) of the methane production rate in the methanation reaction catalysts (catalysts after reduction) of Examples 15 to 24 and Comparative Examples 9 to 12.
FIG. 6 shows a graph illustrating the correlation between the mole ratio (Ni/Ti) of the Ni atom with respect to the Ti atom and the ratio (k/k₀) of the methane production rate in the methanation reaction catalysts (catalysts after reduction) of Examples 25 to 34 and Comparative Examples 13 to 16.
FIG. 7 shows a graph illustrating the correlation between the mole ratio (Zr/Ti) of the Zr atom with respect to the Ti atom and the ratio (k/k₀) of the methane production rate in the methanation reaction catalysts (catalysts after reduction) of Examples 25 to 34 and Comparative Examples 13 to 16.
FIG. 8 shows a graph illustrating the correlation between the mole ratio (Ni/Ce) of the Ni atom with respect to the Ce atom and the ratio (k/k₀) of the methane production rate in the methanation reaction catalysts (catalysts after reduction) of Examples 35 to 44 and Comparative Examples 17 to 20.
FIG. 9 shows a graph illustrating the correlation between the mole ratio (Zr/Ce) of the Zr atom with respect to the Ce atom and the ratio (k/k₀) of the methane production rate in the methanation reaction catalysts (catalysts after reduction) of Examples 35 to 44 and Comparative Examples 17 to 20.

### DESCRIPTION OF EMBODIMENTS

### (First Embodiment)

### (1) Methanation Reaction Catalyst

A methanation reaction catalyst is a methanation reaction catalyst for subjecting CO and/or CO₂ to methanation reaction with hydrogen, and contains a stabilized zirconia support, Ni supported by the stabilized zirconia support, and an inorganic oxide.

In the stabilized zirconia support, a stabilizing element and Ni are solid-solved in a tetragonal and/or cubic crystal structure (unit lattice) mainly composed of Zr. The crystal structure of the stabilized zirconia support is mainly (basically) composed of Zr, and a Zr ion (Zr⁴⁺) is mainly disposed in a plurality of lattice points of the crystal structure of the stabilized zirconia support.

The stabilizing element stabilizes the crystal structure of the stabilized zirconia support so as to be tetragonal and/or cubic. The stabilizing element is at least one of element selected from the group consisting of Y, La, Ce, Pr, Nd, Sm, Gd, Dy, Ca, Mg, Mn, Fe, and Co. Of the stabilizing elements, preferably, Sm, Ca, and Mn are used, more preferably, Ca and Mn are used.

When the stabilizing element and Ni are solid-solved in the stabilized zirconia support, in a part of lattice point of the plurality of lattice points of the crystal structure, the Zr ion is replaced with any one of the above-described stabilizing element ion and the Ni ion.

That is, the solid-solving of the stabilizing element in the stabilized zirconia support means the replacement of the Zr ion disposed in the lattice point of the crystal structure with the above-described stabilizing element ion. Also, the solid-solving of Ni in the stabilized zirconia support means the replacement of the Zr ion disposed in the lattice point of the crystal structure with the Ni ion. Thus, any one of the Zr ion, the above-described stabilizing element ion, and the Ni ion is disposed in the plurality of lattice points of the stabilized zirconia support.

The stabilized zirconia support contains Zr, the above-described stabilizing element, Ni, and O. Preferably, the stabilized zirconia support consists of Zr, the above-described stabilizing element, Ni, and O. To be more specific, the stabilized zirconia support is represented by the following General Formula (1).
General Formula (1):

Zr⁴⁺_{1-(x+y)}M^{α+}ₓNi²⁺_{y}O_{2-((2-α/2)x+y)} (1)

(in the formula (1), x and y are below 1, and x + y is below 1; M is at least one of stabilizing element selected from the group consisting of Y, La, Ce, Pr, Nd, Sm, Gd, Dy, Ca, Mg, Mn, Fe, and Co; and α is a valence of the stabilizing element)

In General Formula (1), x is, for example, 0.133 or more and below 1, and preferably, 0.248 or less. In General Formula (1), y is, for example, 0.010 or more and below 1, and preferably 0.050 or less.

The stabilized zirconia support has an oxygen vacancy. The stabilizing element and/or Ni are/is solid-solved in the stabilized zirconia support, and the stabilizing element ion and/or the Ni ion having a valence of 3 or less (divalent or trivalent) are/is replaced with the Zr ion, so that the oxygen vacancy is formed. These stabilized zirconia supports may be contained alone or in combination of two or more in the methanation reaction catalyst.

Ni may be NiO or may be Ni in a metal state, and in view of catalytic activity, preferably, Ni in a metal state.

As described above, Ni is solid-solved in the stabilized zirconia support, and supported by the stabilized zirconia support. Thus, the methanation reaction catalyst contains Zr that constitutes the stabilized zirconia support, the stabilizing element that is solid-solved in the stabilized zirconia support, Ni that is solid-solved in the stabilized zirconia support, Ni that is supported by the stabilized zirconia support, and the inorganic oxide. Preferably, the methanation reaction catalyst consists of Zr that constitutes the stabilized zirconia support, the stabilizing element that is solid-solved in the stabilized zirconia support, Ni that is solid-solved in the stabilized zirconia support, Ni that is supported by the stabilized zirconia support, and the inorganic oxide.

The inorganic oxide is uniformly dispersed in the methanation reaction catalyst, and acts so as to form a space between a plurality of particles of the stabilized zirconia support that supports Ni.

The inorganic oxide is at least one of inorganic oxide selected from the group consisting of silica (SiO₂), alumina (Al₂O₃), titania (TiO₂), and ceria (CeO₂).

Examples of the silica include quartz, cristobalite, tridymite, and coesite. Preferably, quartz is used.

Examples of the alumina include α-alumina, θ-alumina, and γ-alumina. Preferably, γ-alumina is used.

Examples of the titania include rutile-type titania, anatase-type titania, and brookite-type titania. Preferably, rutile type titania is used.

An example of the ceria includes fluorite-type ceria.

Of the inorganic oxides, preferably, silica is used. The inorganic oxides may be contained alone or in combination of two or more in the methanation reaction catalyst.

In the following, when the Ni content is represented, the total sum of Ni that is supported by the stabilized zirconia support and Ni that is solid-solved in the stabilized zirconia support is represented as Ni. Each of the Si atom, the Al atom, the Ti atom, and the Ce atom contained in the inorganic oxide is defined as an inorganic element, and the total sum of the inorganic element is represented as the total inorganic element.

In the methanation reaction catalyst, the atom ratio (= Zr/(Zr + stabilizing element + Ni + total inorganic element) × 100) of Zr with respect to the total sum (hereinafter, referred to as the total sum of each of the atoms) of Zr, the stabilizing element, Ni, and the total inorganic element is, for example, 8.5 atom% or more, preferably 15.0 atom% or more, more preferably 23.0 atom% or more, and for example, 70 atom% or less, preferably 60 atom% or less, more preferably 55.0 atom% or less. The atom ratio of each of the atoms in the methanation reaction catalyst is calculated from the charged amount of the material component (zirconia and/or salt of Zr, salt of stabilizing element, salt of Ni, and inorganic oxide) used in a method for producing a methanation reaction catalyst to be described later.

In the methanation reaction catalyst, the atom ratio (= stabilizing element/(Zr + stabilizing element + Ni + total inorganic element) × 100) of the stabilizing element with respect to the total sum of each of the atoms is, for example, 1.0 atom% or more, preferably 1.5 atom% or more, and for example, 20 atom% or less, preferably 7.5 atom% or less.

In the methanation reaction catalyst, the atom ratio (= Ni/(Zr + stabilizing element + Ni + total inorganic element) × 100) of Ni with respect to the total sum of each of the atoms is, for example, 10 atom% or more, preferably 15 atom% or more, more preferably 23.0 atom% or more, and for example, 70 atom% or less, preferably 55.0 atom% or less.

In the methanation reaction catalyst, the atom ratio (= inorganic element /(Zr + stabilizing element + Ni + total inorganic element) × 100) of the total inorganic element with respect to the total sum of each of the atoms is, for example, 2.0 atom% or more, preferably 3.0 atom% or more, more preferably 10 atom% or more, and for example, 75 atom% or less, preferably 50 atom% or less.

When the inorganic oxide contains the silica, the mole ratio of the Ni atom with respect to the Si atom is, for example, 0.20 or more, preferably 0.35 or more, more preferably 0.70 or more, and for example, 10.0 or less, preferably 5.0 or less, more preferably 3.0 or less.

When the inorganic oxide contains the silica, the mole ratio of the Zr atom with respect to the Si atom is, for example, 0.10 or more, preferably 0.20 or more, and for example, 4.5 or less, preferably 3.0 or less, more preferably 1.5 or less.

When the inorganic oxide contains the alumina, the mole ratio of the Ni atom with respect to the Al atom is, for example, 0.20 or more, preferably 0.30 or more, more preferably 1.0 or more, and for example, 7.0 or less, preferably 5.0 or less, more preferably 2.5 or less.

When the inorganic oxide contains the alumina, the mole ratio of the Zr atom with respect to the Al atom is, for example, 0.10 or more, preferably 0.15 or more, more preferably 0.50 or more, and for example, 4.0 or less, preferably 2.5 or less, more preferably 1.5 or less.

When the inorganic oxide contains the titania, the mole ratio of the Ni atom with respect to the Ti atom is, for example, 0.30 or more, preferably 0.40 or more, more preferably 1.0 or more, and for example, 10.5 or less, preferably 8.0 or less, more preferably 4.0 or less.

When the inorganic oxide contains the titania, the mole ratio of the Zr atom with respect to the Ti atom is, for example, 0.15 or more, preferably 0.20 or more, more preferably 0.50 or more, and for example, 6.0 or less, preferably 4.0 or less, more preferably 2.0 or less.

When the inorganic oxide contains the ceria, the mole ratio of the Ni atom with respect to the Ce atom is, for example, 0.70 or more, preferably 1.0 or more, more preferably 2.0 or more, and for example, 25.0 or less, preferably 15.0 or less, more preferably 9.0 or less.

When the inorganic oxide contains the ceria, the mole ratio of the Zr atom with respect to the Ce atom is, for example, 0.30 or more, preferably 0.50 or more, more preferably 1.0 or more, and for example, 12.0 or less, preferably 7.0 or less, more preferably 5.0 or less.

When the mole ratio of the Ni atom with respect to the inorganic element (at least any one of the Si atom, the Al atom, the Ti atom, and the Ce atom) is below the above-described lower limit, the content ratio of Ni that is the catalytically active component can be surely ensured, and the improvement of the methane production rate of the methanation reaction catalyst can be achieved. When the mole ratio of the Ni atom with respect to the inorganic element is above the above-described upper limit, the improvement of the fine pore volume in the methanation reaction catalyst can be achieved, and the improvement of the methane production rate of the methanation reaction catalyst can be achieved.

In a case where two or more of the inorganic oxides are contained in the methanation reaction catalyst, when the mole ratio of the Ni atom with respect to at least one of the inorganic element contained in the inorganic elements is within the above-described range, it is included in the range of the present invention. Meanwhile, in a case where two or more of the inorganic oxides are contained in the methanation reaction catalyst, when the mole ratio of the Ni atom with respect to any of the inorganic element contained in the inorganic elements is the outside the above-described range, it is the outside the range of the present invention.

When the mole ratio of the Zr atom with respect to the inorganic element is within the above-described range, the improvement of the fine pore volume in the methanation reaction catalyst can be further achieved, and the improvement of the methane production rate of the methanation reaction catalyst can be further achieved.

The shape of the methanation reaction catalyst is not particularly limited, and preferably, has a particle shape. When the methanation reaction catalyst has a particle shape, the average secondary particle size of the methanation reaction catalyst, is for example, 1.0 µm or more, preferably 10 µm or more, and for example, 200 µm or less, preferably 150 µm or less. The average secondary particle size is measured in accordance with an electron microscope method (JIS H7803: 2005).

The methanation reaction catalyst has a plurality of fine pores.

Of the plurality of fine pores, the total sum of the volume of the fine pore having a fine pore diameter of 2 nm to 12 nm (hereinafter, referred to as 2-12 nm fine pore) is, for example, 0.050 cm³/g or more, preferably 0.070 cm³/g or more per unit mass of the methanation reaction catalyst. The larger the total sum of the volume the 2-12 nm fine pore is, the better it is. The total sum of the volume of the 2-12 nm fine pore is, for example, 0.220 cm³/g or less, preferably 0.180 cm³/g or less. The total sum of the volume of the 2-12 nm fine pore is measured in conformity with Examples to be described later.

When the total sum of the volume of the 2-12 nm fine pore is within the above-described range, the improvement of the specific surface area of the methanation reaction catalyst can be achieved, and the improvement of the surface-exposed amount of Ni can be achieved.

The specific surface area (BET specific surface area) of the methanation reaction catalyst is, for example, 50 m²·g⁻¹ or more, preferably 70 m²·g⁻¹ or more. The larger the specific surface area (BET specific surface area) of the methanation reaction catalyst is, the better it is. The specific surface area (BET specific surface area) of the methanation reaction catalyst is, for example, 200 m²·g⁻¹ or less, preferably 150 m²·g⁻¹ or less. The specific surface area of the methanation reaction catalyst is measured in accordance with a BET method (JIS Z8830: 2013).

### (2) Production Method of Methanation Reaction Catalyst

The methanation reaction catalyst is a calcined product of a wet mixture of zirconia and/or a salt of Zr, a salt of a stabilizing element, a salt of Ni, and an inorganic oxide.

Next, one embodiment of the method for producing a methanation reaction catalyst is described.

The method for producing a methanation reaction catalyst includes a step (mixing step) of wet-mixing a material component to prepare a wet mixture and a step (calcining step) of calcining the wet mixture, and if necessary, further includes a step (reducing step) of reducing NiO to Ni.

In the mixing step, as the material component, zirconia (ZrO₂) and/or the salt of Zr, the salt of the above-described stabilizing element, the salt of Ni, and the above-described inorganic oxide are wet-mixed so that the atom ratio of each of the atoms (Zr, stabilizing element, Ni, and inorganic element) is, for example, within the above-described range.

An example of the zirconia includes a low crystalline ZrO₂ fine particle.

Examples of the salt of Zr include nitrate of Zr (for example, zirconium nitrate (Zr(NO₃)₄), zirconium nitrate oxide (ZrO (NO₃)₂, or the like), hydrochloride of Zr (for example, zirconium chloride oxide (ZrCl₂O) or the like), and acetate of Zr (for example, zirconium acetate oxide (ZrO(C₂H₃O₂)₂) or the like). Theses salts of Zr can be used alone or in combination of two or more.

Of zirconia and the salts of Zr, preferably, an acetate of Zr is used, more preferably, a zirconium acetate oxide is used.

Examples of the salt of the stabilizing element include nitrate of the stabilizing element (for example, samarium nitrate (Sm(NO₃)₃), calcium nitrate (Ca(NO₃)₂), manganese nitrate (Mn(NO₃)₂), iron nitrate (Fe(NO₃)₃), cobalt nitrate (Co(NO₃)₂), or the like) and chloride of the stabilizing nitrate (for example, samarium chloride (SmCl₃), calcium chloride (CaCl₂), manganese chloride (MnCl₂), iron chloride (FeCl₃), cobalt chloride (CoCl₂), or the like). These salts of the stabilizing element can be used alone or in combination of two or more. As the salt of the stabilizing element, a commercially available product can be also used.

Of the salts of the stabilizing element, preferably, a nitrate of the stabilizing element is used, more preferably, a samarium nitrate and a calcium nitrate are used.

Examples of the salt of Ni include nitrate of Ni (for example, nickel nitrate (Ni(NO₃)₂) or the like) and chloride of Ni (for example, nickel chloride (NiCl₂) or the like). These salts of Ni can be used alone or in combination of two or more.

Of the salt of Ni, preferably, a nitrate of Ni is used, more preferably, a nickel nitrate is used.

To wet-mix the material component, for example, the salt of the stabilizing element, the salt of Ni, and the inorganic oxide are added to the hydrosol of zirconia and/or an aqueous solution of the salt of Zr so that the atom ratio of each of the atoms (Zr, stabilizing element, Ni, and inorganic element) is within the above-described range to be stirred and mixed.

To be more specific, the salt of the stabilizing element is added to the hydrosol of zirconia and/or the aqueous solution of the salt of Zr to be stirred and mixed (wet-mixed) until a uniform slurry.

Next, the salt of Ni (preferably, an aqueous solution of the salt of Ni) is added to the slurry to be stirred and mixed (wet-mixed) until a uniform mixed solution.

Next, the inorganic oxide (preferably, hydrosol of the inorganic oxide) is added to the mixed solution to be stirred (wet-mixed) until a uniform solution, so that a wet mixture is prepared.

In this manner, the wet mixture containing zirconia and/or the salt of Zr, the salt of the stabilizing element, the salt of Ni, and the inorganic oxide is prepared.

Next, the wet mixture is, for example, heated with a constant-temperature drying oven, so that excessive moisture is volatilized.

The heating temperature of the wet mixture is, for example, 100°C or more, preferably 150°C or more, and for example, 200°C or less, preferably 170°C or less. The heating time of the wet mixture is, for example, 30 minutes or more, preferably 1 hour or more, and for example, 10 hours or less, preferably 3 hours or less.

Next, the wet mixture is calcined with, for example, a heating oven such as electric oven in the calcining step.

The calcining temperature is, for example, 400°C or more, preferably 500°C or more, more preferably 600°C or more, and for example, 800°C or less, preferably 750°C or less, more preferably 700°C or less.

When the calcining temperature is within the above-described range, the crystal structure of the stabilized zirconia support can be surely brought into the tetragonal and/or cubic crystal-type.

The calcining time is, for example, 1 hour or more, preferably 5 hours or more, and for example, 24 hours or less, preferably 10 hours or less.

In this manner, the wet mixture is calcined, the stabilized zirconia support represented by the above-described General Formula (1) is formed, and as represented by the following General Formula (2), the nickel oxide is supported by stabilized zirconia support.
General Formula (2):

NiO/Zr⁴⁺_{1-(x+y)}M^{α+}ₓNi²⁺_{y}O_{2-((2-α/2)x+y)} (2)

(in the formula (2), x and y have the same range as that of x and y in the General Formula (1); M represents the same stabilizing element as that of M in the General Formula (1); and α has the same range as that of the General Formula (1))

As described above, the methanation reaction catalyst that is the calcined product of the wet mixture is prepared.

The methanation reaction catalyst is a catalyst before reduction before NiO is reduced to Ni, and contains the stabilized zirconia support in which the nickel oxide represented by the above-described General Formula (2) is supported, and the inorganic oxide. In the methanation reaction catalyst, the stabilized zirconia support of the above-described General Formula (2) and the inorganic oxide are uniformly dispersed each other.

The content ratio of the inorganic oxide with respect to the catalyst before reduction (the total sum of the calcined product of the wet mixture of zirconia and/or the salt of Zr, the salt of the stabilizing element, the salt of Ni, and the inorganic oxide) is, for example, 5 mass% or more, preferably 10 mass% or more, and for example, 62 mass% or less, preferably 45 mass% or less.

When the content ratio of the inorganic oxide is within the above-described range, the improvement of the dispersibility of Ni can be stably achieved, and the improvement of the fine pore volume in the methanation reaction catalyst can be stably achieved.

The total sum of the volume of the 2-12 nm fine pore in the catalyst before reduction is the same as that of the above-described range.

Next, in the reducing step, the catalyst before reduction is subjected to reduction treatment by a hydrogen current.

To be more specific, the catalyst before reduction fills the inside of a predetermined reaction tube, and thereafter, the catalyst before reduction is heated with, for example, a heater such as tubular electric furnace so that the inside of the reaction tube reaches the following reduction temperature, and the hydrogen is circulated in the reaction tube.

The reduction temperature is, for example, 200°C or more, preferably 300°C or more, and for example, 600°C or less, preferably 500°C or less. The reduction time is, for example, 2 hours or more, preferably 5 hours or more, and for example, 10 hours or less.

In this manner, the nickel oxide supported by the stabilized zirconia support is reduced to nickel in a metal state as represented by the following General Formula (3). General Formula (3):

Ni/Zr⁴⁺_{1-(x+y)}M^{α+}ₓNi²⁺_{y}O_{2-((2-α/2)x+y)} (3)

As described above, the methanation reaction catalyst (catalyst after reduction) containing the stabilized zirconia support supported by the nickel in a metal state represented by the above-described General Formula (3), and the inorganic oxide is prepared. In the catalyst after reduction, the stabilized zirconia support of the above-described General Formula (3) and the inorganic oxide are uniformly dispersed each other.

Each of the range of the mole ratio of the Ni atom with respect to the Si atom and the range of the mole ratio of the Zr atom with respect to the Si atom in the catalyst after reduction is the same as the above-described range. The range of the total sum of the volume of the 2-12 nm fine pore per unit mass of the catalyst after reduction is the same as the above-described range.

### (3) Method for Producing Methane

Next, a method for producing methane by using the above-described methanation reaction catalyst is described.

To produce the methane by the methanation reaction catalyst, the methanation reaction catalyst is brought into contact with a mixed gas containing CO and/or CO₂, and a hydrogen gas at the reaction temperature of 200°C or more.

To be more specific, the methanation reaction catalyst fills a predetermined reaction tube. Then, the reaction tube is retained at the following reaction temperature under a normal pressure, and the mixed gas is supplied to the reaction tube.

The reaction temperature is, for example, 200°C or more, preferably 250°C or more, more preferably 300°C or more, and for example, 500°C or less, preferably 400°C or less.

When the mixed gas contains CO₂ and the hydrogen gas, the mole ratio of CO₂ with respect to the hydrogen gas is 1 to 4, and when the mixed gas contains CO and the hydrogen gas, the mole ratio of CO with respect to the hydrogen gas is 1 to 3.

The flow rate of the mixed gas per 1 g of the methanation reaction catalyst is, for example, 1000 L·h⁻¹·g⁻¹ or more, preferably 2000 L·h⁻¹·g⁻¹ or more, and for example, 5000 L·h⁻¹·g⁻¹ or less, preferably 4000 L·h⁻¹·g⁻¹ or less.

In this manner, when the methanation reaction catalyst is brought into contact with the mixed gas, even in a case where NiO is supported by the stabilized zirconia support, NiO is reduced to Ni in a metal state by the hydrogen in the mixed gas.

The oxygen vacancy of the stabilized zirconia support attracts the oxygen atom of CO and/or CO₂, and Ni in a metal state supported by the stabilized zirconia support attracts the hydrogen, so that CO and/or CO₂ and the hydrogen efficiently react to each other on the surface of the methanation reaction catalyst, thereby producing the methane.

At this time, the methane production rate per 1 g of the methanation reaction catalyst at 250°C is, for example, 0.070 mmol·s⁻¹·g⁻¹ or more, preferably 0.080 mmol·s⁻¹·g⁻¹ or more. The faster the methane production rate per 1 g of the methanation reaction catalyst at 250°C is, the better it is. The methane production rate per 1 g of the methanation reaction catalyst at 250°C is, for example, 0.125 mmol·s⁻¹·g⁻¹ or less.

### (4) Function and Effect

The methanation reaction catalyst is the calcined product of the wet mixture of zirconia and/or the salt of Zr, the salt of the stabilizing element, the salt of Ni, and the inorganic oxide.

When the inorganic oxide contains the silica, the mole ratio of the Ni atom with respect to the Si atom is 0.20 or more and 10.0 or less; when the inorganic oxide contains the alumina, the mole ratio of the Ni atom with respect to the Al atom is 0.20 or more and 7.0 or less; when the inorganic oxide contains the titania, the mole ratio of the Ni atom with respect to the Ti atom is 0.30 or more and 10.5 or less; and when the inorganic oxide contains the ceria, the mole ratio of the Ni atom with respect to the Ce atom is 0.70 or more and 25.0 or less.

Thus, the inorganic oxide acts so as to form a space between the plurality of Ni atoms that are a catalytically active component, and suppresses the aggregation of Ni. As a result, the improvement of the fine pore volume in the methanation reaction catalyst can be achieved, and the improvement of the dispersibility of Ni can be achieved. In this manner, the improvement of the specific surface area of the methanation reaction catalyst can be achieved, and an increase in the surface-exposed amount of Ni can be achieved. Thus, the improvement of the catalytic activity of the methanation reaction catalyst can be achieved, and the improvement of the methane production rate can be achieved.

Meanwhile, when the mole ratio of the Ni atom with respect to the inorganic element (each of the Si atom, the Al atom, the Ti atom, and the Ce atom) contained in the inorganic oxide is outside the above-described range, the above-described function and effect cannot be achieved.

To be specific, when the mole ratio of the Ni atom with respect to the inorganic element contained in the inorganic oxide is above the above-described upper limit, for example, as described in Patent Document 1, when the mole ratio of the Ni atom with respect to the Si atom is 11.8 (= 60.2/5.1), the content ratio of the inorganic oxide is insufficient, and the space cannot be sufficiently formed between the plurality of Ni atoms, and the improvement of the fine pore volume in the methanation reaction catalyst cannot be sufficiently achieved.

Thus, the improvement of the catalytic activity of the methanation reaction catalyst cannot be sufficiently achieved, and the improvement of the methane production rate cannot be sufficiently achieved.

Also, when the mole ratio of the Ni atom with respect to the inorganic element contained in the inorganic oxide is below the above-described lower limit, the content ratio of Ni that is the catalytically active component cannot be sufficiently ensured, and the methane production rate is reduced. This is clear by Examples and Comparative Examples to be described later.

In the methanation reaction catalyst, preferably, when the inorganic oxide contains the silica, the mole ratio of the Zr atom with respect to the Si atom is 0.10 or more and 4.5 or less; when the inorganic oxide contains the alumina, the mole ratio of the Zr atom with respect to the Al atom is 0.10 or more and 4.0 or less; when the inorganic oxide contains the titania, the mole ratio of the Zr atom with respect to the Ti atom is 0.15 or more and 6.0 or less; and when the inorganic oxide contains the ceria, the mole ratio of the Zr atom with respect to the Ce atom is 0.30 or more and 12.0 or less.

Thus, the inorganic oxide acts so as to form a space between the plurality of particles of the stabilized zirconia support, and the improvement of the fine pore volume in the methanation reaction catalyst can be further achieved. In this manner, the improvement of the specific surface area of the methanation reaction catalyst can be further achieved, and the improvement of the methane production rate can be further achieved.

The content ratio of the inorganic oxide with respect to the catalyst before reduction (total sum of the calcined product of the wet mixture) is preferably 5 mass% or more and 62 mass% or less. Thus, the improvement of the dispersibility of Ni can be stably achieved, and the improvement of the fine pore volume in the methanation reaction catalyst can be stably achieved.

In the methanation reaction catalyst (catalyst before reaction), preferably, the total sum of the volume of the fine pore having a fine pore diameter of 2 nm to 12 nm is 0.050 cm³/g or more per unit mass. Thus, an increase in the surface-exposed amount of Ni can be further achieved, and the improvement of the catalytic activity of the methanation reaction catalyst can be further achieved.

Also, zirconia and/or the salt of Zr, the inorganic oxide, the salt of the stabilizing element, and the salt of Ni are wet-mixed so that the mole ratio of the Ni atom with respect to at least any one of the Si atom, the Al atom, the Ti atom, and the Ce atom is within the above-described range to prepare a wet mixture, and thereafter, the wet mixture is calcined at 400°C or more and 800°C or less, thereby producing the methanation reaction catalyst.

Thus, even by the easy method, the improvement of the catalytic activity of the methanation reaction catalyst can be achieved, and the methanation reaction catalyst that is capable of achieving the improvement of the methane production rate can be produced. Examples

Next, the present invention is further described based on Examples. The present invention is however not limited by these Examples. The specific numerical values in mixing ratio (content ratio), property value, and parameter used in the following description can be replaced with upper limit values (numerical values defined as "or less" or "below") or lower limit values (numerical values defined as "or more" or "above") of corresponding numerical values in mixing ratio (content ratio), property value, and parameter described in the above-described "DESCRIPTION OF EMBODIMENTS".

A measurement method of the methane production rate described in the following is described as follows.

### <Methane Production Rate>

A methanation reaction catalyst (catalyst after reduction) filled a reaction tube (SUS304 tube, inner diameter of 15 mm × height of 100 mm).

Next, the reaction tube was retained at 250°C (reaction temperature) under a normal pressure, and a material gas (mixed gas) containing carbon dioxide, hydrogen, and nitrogen was supplied to the reaction tube to be brought into contact with the methanation reaction catalyst.

In the material gas, the ratio of hydrogen/carbon dioxide was 4 (mole ratio), and the nitrogen was 5% by volume. The flow rate of the material gas was 1.0 L/min.

The reaction gas that was brought into contact with the methanation reaction catalyst to be flowed out of the reaction tube was analyzed with a thermal conductivity detecting (TCD)-type gas chromatography. The reaction gas contained only the unreacted hydrogen, the unreacted carbon dioxide, and the methane that was a product.

The methane production rate (unit: mmol·s⁻¹·g⁻¹) per 1 g of the catalyst was calculated from the ratio of the amount of the hydrogen and the carbon dioxide supplied to the reaction tube to the amount of the unreacted hydrogen and the unreacted carbon dioxide flowed out of the reaction tube.

### (Examples 1 to 9 and Comparative Examples 1 to 3)

A crystal of samarium nitrate hexahydrate (1.97 g) was added to 15.00 g of hydrosol of zirconia (trade name: Zr30AH, manufactured by Nissan Chemical Corporation, ZrO₂: 30 wt.%, pH = 4.0) to be stirred (wet-mixed) until a uniform slurry (sludge in a cream state).

Next, an aqueous solution of nickel nitrate in which 19.81 g of nickel nitrate hexahydrate was dissolved in 20 mL of pure water was added to the slurry to be stirred (wet-mixed) until a uniform mixed solution.

Next, a silica sol (trade name: SNOWTEX OS, manufactured by Nissan Chemical Corporation, SiO₂: 20 wt.%, ph = 2.0 to 4.0) was added to the mixed solution so that the atom ratio [Si/(Ni + Zr + Sm + Si)] of Si was the value shown in the following Table 1, and the resulting mixture was stirred (wet-mixed) until a uniform solution, thereby preparing a wet mixture. The wet mixture was a mixture of zirconia, samarium nitrate, nickel nitrate, and silica except for Comparative Example 1. In Comparative Example 1, the silica sol was not added to the mixed solution, and the wet mixture did not contain the silica.

Next, the wet mixture was put into a constant-temperature drying oven retained at 170°C to be dried for 2 hours. In this manner, excessive moisture was volatilized from the wet mixture.

Next, the wet mixture after drying was calcined at 500°C for 8 hours, so that a methanation reaction catalyst (hereinafter, referred to as a catalyst before reduction) was prepared. The catalyst before reduction included a stabilized zirconia support in which Ni and Sm were solid-solved, NiO supported by the stabilized zirconia support, and silica. The catalyst before reduction of Comparative Example 1 did not include the silica.

The fine pore distribution of each of the catalysts before reduction was measured by a nitrogen absorption method. The total sum V of the volume of a fine pore having a fine pore diameter of 2 nm to 12 nm (hereinafter, referred to as a 2-12 nm fine pore) was calculated from the obtained fine pore distribution. The results are shown in Table 1. In Table 1, the ratio (V/V₀) of the total sum V of the volume of the 2-12 nm fine pore in each of the catalysts before reduction with respect to the total sum V₀ of the volume of the 2-12 nm fine pore in the catalyst before reduction of Comparative Example 1 is shown.

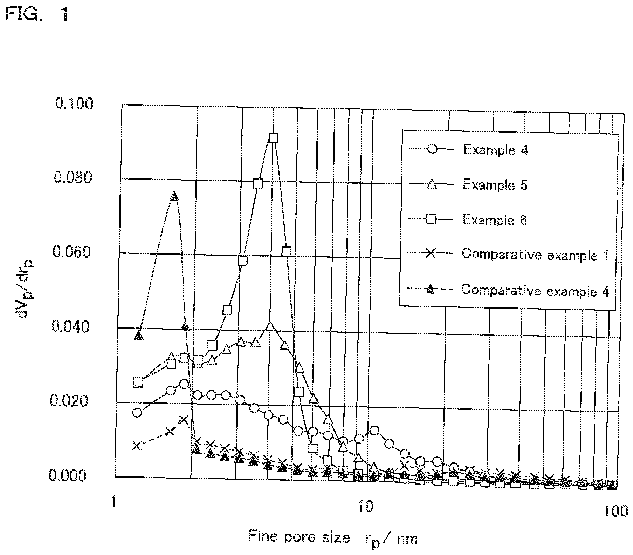

The fine pore distribution of each of Examples 4 to 6 and Comparative Example 1 is shown in FIG. 1.

Next, 20 mg of catalyst before reduction and 5 g of reaction inactive alumina (γ-Al₂O₃) were uniformly mixed to be fixed in a quartz tube (reaction tube) having an inner diameter of 15 mm with a quartz wool. The reaction tube was disposed at the inside of the electric furnace to be heated so that the temperature at the inside of the reaction tube was 300°C, and hydrogen was circulated at the inside of the reaction tube to be retained for 2 hours. In this manner, NiO contained in the catalyst before reduction was reduced to Ni in a metal state.

As described above, the methanation reaction catalyst (catalyst after reduction) containing the stabilizing zirconia in which Ni and Sm were solid-solved, the silica, and Ni in a metal state was prepared. The methanation reaction catalyst (catalyst after reduction) of Comparative Example 1 did not contain the silica.

Next, the methane production rate k of each of the methanation reaction catalysts (catalysts after reduction) was measured. The results are shown in Table 1. In Table 1, the ratio (k/k₀) of the methane production rate k of each of the methanation reaction catalysts with respect to the methane production rate k₀ of the methanation reaction catalyst of Comparative Example 1 is shown.

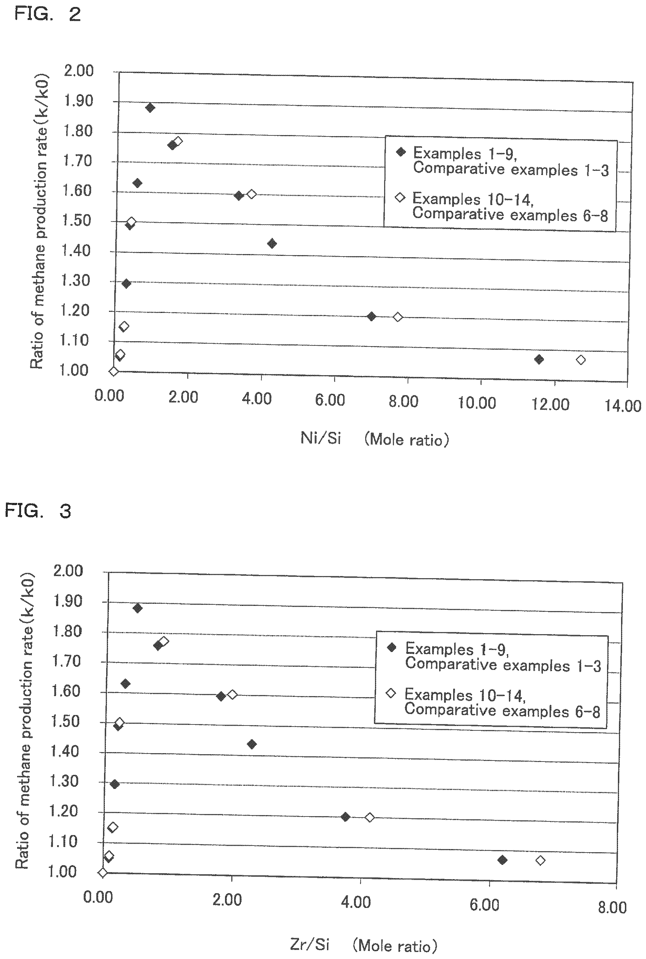

The correlation between the mole ratio (Ni/Si) of the Ni atom with respect to the Si atom and k/k₀ in each of the methanation reaction catalysts (catalysts after reduction) is shown in FIG. 2, and the correlation between the mole ratio (Zr/Si) of the Zr atom with respect to the Si atom and k/k₀ in each of the methanation reaction catalysts (catalysts after reduction) is shown in FIG. 3.

### (Comparative Example 4)

The catalyst before reduction was produced in the same manner as that of Comparative Example 1. The above-described silica sol was calcined at 500°C for 8 hours, thereby preparing a silica powder.

Next, the catalyst before reduction of Comparative Example 1 and the silica powder were dry-mixed so that the atom ratio [Si/(Ni + Zr + Sm + Si)] of Si was 29.8 atom%, thereby preparing a dry mixture. The fine pore distribution of the dry mixture was measured by a nitrogen absorption method. The results are shown in Table 1 and FIG. 1.

Next, the dry mixture was reduced under the same conditions as those of Example 1, so that the methanation reaction catalyst (catalyst after reduction) was produced. Then, the methane production rate k of the methanation reaction catalyst (catalyst after reduction) was measured. The results are shown in Table 1.

### (Comparative Example 5)

The catalyst before reduction was produced in the same manner as that of Comparative Example 1. Then, the catalyst before reduction and the above-described silica sol were stirred and mixed so that the atom ratio [Si/(Ni + Zr + Sm + Si)] of Si was 29.8 atom%, thereby preparing a suspension solution.

Next, the suspension solution was calcined at 500°C for 8 hours, so that the catalyst before reduction was produced. The fine pore distribution of the catalyst before reduction was measured by a nitrogen absorption method. The results are shown in Table 1.

Next, the catalyst before reduction was reduced under the same conditions as those of Example 1, so that the methanation reaction catalyst (catalyst after reduction) was produced. Then, the methane production rate k of the methanation reaction catalyst (catalyst after reduction) was measured. The results are shown in Table 1.

### (Examples 10 to 14 and Comparative Examples 6 to 8)

The methanation reaction catalysts (catalysts after reduction) were produced in the same manner as that of Example 1, except that 2.15 g of calcium nitrate tetrahydrate was used instead of 1.97 g of samarium nitrate hexahydrate, and the above-described silica sol was added to the mixed solution so that the atom ratio [Si/(Ni + Zr + Ca + Si)] of Si was the value shown by the following Table 2. In Comparative Example 6, the silica sol was not added to the mixed solution.

The total sum V of the volume of the 2-12 nm fine pore in each of the catalysts before reduction, and the ratio (V/V₀) of the total sum V of the volume of the 2-12 nm fine pore in each of the catalysts before reduction with respect to the total sum V₀ of the volume of the 2-12 nm fine pore in the catalyst before reduction of Comparative Example 6 is shown in Table 2.

The methane production rate k of each of the methanation reaction catalysts (catalysts after reduction), and the ratio (k/k₀) of the methane production rate k of each of the methanation reaction catalysts with respect to the methane production rate k₀ of the methanation reaction catalyst of Comparative Example 6 is shown in Table 2.

The correlation between the mole ratio (Ni/Si) of the Ni atom with respect to the Si atom and k/k₀ in each of the methanation reaction catalysts (catalysts after reduction) is shown in FIG. 2, and the correlation between the mole ratio (Zr/Si) of the Zr atom with respect to the Si atom and k/k₀ in each of the methanation reaction catalysts (catalysts after reduction) is shown in FIG.

### [Table 1]

**Table 1**

| No. | Composition of Methanation Reaction Catalyst | | | | Content Ratio (wt.%) of SiO₂ in Catalyst before Reduction | Mole Ratio | | Fine Pore Volume | | Methane Production Rate | |
|---|---|---|---|---|---|---|---|---|---|---|---|
| | Atom Ratio (at.%) | | | | | | | V[2-12nm] (cm³/g) | V/V0 | k (mmol/(s·g)) | k/k0 |
| | Ni | Zr | Sm | Si | | Ni/Si | Zr/Si | | | | |
| Comp. Ex. 1 | 62.5 | 33.5 | 4.1 | 0 | 0 | 0.00 | 0.00 | 0.035 | 1.00 | 0.064 | 1.00 |
| Comp. Ex. 2 | 59.3 | 31.8 | 3.9 | 5.1 | 3.3 | 11.56 | 6.20 | 0.049 | 1.39 | 0.068 | 1.07 |
| Ex. 1 | 57.3 | 30.7 | 3.7 | 8.2 | 5.4 | 6.98 | 3.74 | 0.056 | 1.60 | 0.077 | 1.20 |
| Ex. 2 | 54.4 | 29.2 | 3.5 | 12.9 | 8.5 | 4.23 | 2.27 | 0.069 | 1.96 | 0.092 | 1.44 |
| Ex. 3 | 52.5 | 28.2 | 3.4 | 15.9 | 10.7 | 3.31 | 1.78 | 0.082 | 2.32 | 0.102 | 1.59 |
| Ex. 4 | 43.8 | 23.5 | 2.9 | 29.8 | 21.2 | 1.47 | 0.79 | 0.137 | 3.88 | 0.113 | 1.76 |
| Ex. 5 | 36.1 | 19.4 | 2.4 | 42.1 | 31.5 | 0.86 | 0.46 | 0.187 | 5.32 | 0.120 | 1.88 |
| Ex. 6 | 29.3 | 15.7 | 1.9 | 53.1 | 41.7 | 0.55 | 0.30 | 0.206 | 5.86 | 0.104 | 1.63 |
| Ex. 7 | 23.1 | 12.4 | 1.5 | 63.0 | 51.8 | 0.37 | 0.20 | 0.221 | 6.29 | 0.095 | 1.49 |
| Ex. 8 | 20.3 | 10.9 | 1.3 | 67.5 | 56.8 | 0.30 | 0.16 | 0.222 | 6.30 | 0.083 | 1.29 |
| Ex. 9 | 17.6 | 9.4 | 1.1 | 71.8 | 61.7 | 0.25 | 0.13 | 0.222 | 6.30 | 0.073 | 1.15 |
| Comp. Ex. 3 | 12.6 | 6.7 | 0.8 | 79.9 | 71.5 | 0.16 | 0.08 | 0.222 | 6.31 | 0.067 | 1.05 |
| Comp. Ex. 4 | 43.8 | 23.5 | 2.9 | 29.8 | 21.2 | 1.47 | 0.79 | 0.030 | 0.84 | 0.063 | 0.99 |
| Comp. Ex. 5 | 43.8 | 23.5 | 2.9 | 29.8 | 21.2 | 1.47 | 0.79 | 0.082 | 2.34 | 0.054 | 0.85 |

### [Table 2]

**Table 2**

| No. | Composition of Methanation Reaction Catalyst | | | | Content Ratio (wt.%) of SiO₂ in Catalyst before Reduction | Mole Ratio | | Fine Pore Volume | | Methane Production Rate | |
|---|---|---|---|---|---|---|---|---|---|---|---|
| | Atom Ratio (at.%) | | | | | | | V[2-12nm] (cm³/g) | V/V0 | k (mmol/(s·g)) | k/k0 |
| | Ni | Zr | Ca | Si | | Ni/Si | Zr/Si | | | | |
| Comp. Ex. 6 | 59.9 | 32.1 | 8.0 | 0 | 0 | 0.00 | 0.00 | 0.037 | 1.00 | 0.069 | 1.00 |
| Comp. Ex. 7 | 57.2 | 30.6 | 7.6 | 4.5 | 3.1 | 12.70 | 6.79 | 0.050 | 1.36 | 0.074 | 1.07 |
| Ex. 10 | 55.6 | 29.8 | 7.4 | 7.2 | 5.0 | 7.71 | 4.12 | 0.059 | 1.60 | 0.083 | 1.20 |
| Ex. 11 | 51.5 | 27.5 | 6.9 | 14.1 | 10.0 | 3.65 | 1.95 | 0.085 | 2.33 | 0.110 | 1.60 |
| Ex. 12 | 43.8 | 23.4 | 5.8 | 27.0 | 20.0 | 1.62 | 0.87 | 0.141 | 3.84 | 0.122 | 1.77 |
| Ex. 13 | 24.2 | 12.9 | 3.2 | 59.6 | . 50.0 | 0.41 | 0.22 | 0.227 | 6.21 | 0.103 | 1.50 |
| Ex. 14 | 18.6 | 10.0 | 2.5 | 68.9 | 60.0 | 0.27 | 0.14 | 0.229 | 6.24 | 0.079 | 1.15 |
| Ex. 8 | 13.5 | 7.2 | 1.8 | 177.5 | Comp. 70.0 | 0.17 | 0.09 | 0.229 | 6.25 | 0.073 | 1.06 |

Even in a case where the stabilizing element is any one of Sm and Ca, when the mole ratio (Ni/Si) of the Ni atom with respect to the Si atom is 0.20 or more and 10.0 or less, a significant increase in the total sum of the volume of the 2-12 nm fine pore was recognized, and the significant improvement of the methane production rate was confirmed (ref: Examples 1 to 14).

Meanwhile, in Comparative Examples 2 and 7 in which Ni/Si was above 10.0, an increase in the total sum of the volume of the 2-12 nm fine pore was insufficient, and the methane production rate was not sufficiently improved. Also, in Comparative Examples 3 and 8 in which Ni/Si was below 0.20, the methane production rate was not sufficiently improved.

In Comparative Example 4, it was confirmed that the total sum of the volume of the 2-12 nm fine pore was reduced, and the methane production rate was reduced. In Comparative Example 5, it was confirmed that the methane production rate was reduced, while an increase in the total sum of the volume of the 2-12 nm fine pore was recognized. This is presumably because Ni that was a catalytically active component was covered with the inorganic oxide (silica), and the exposed amount of Ni is reduced.

### (Examples 15 to 19 and Comparative Examples 9 and 10)

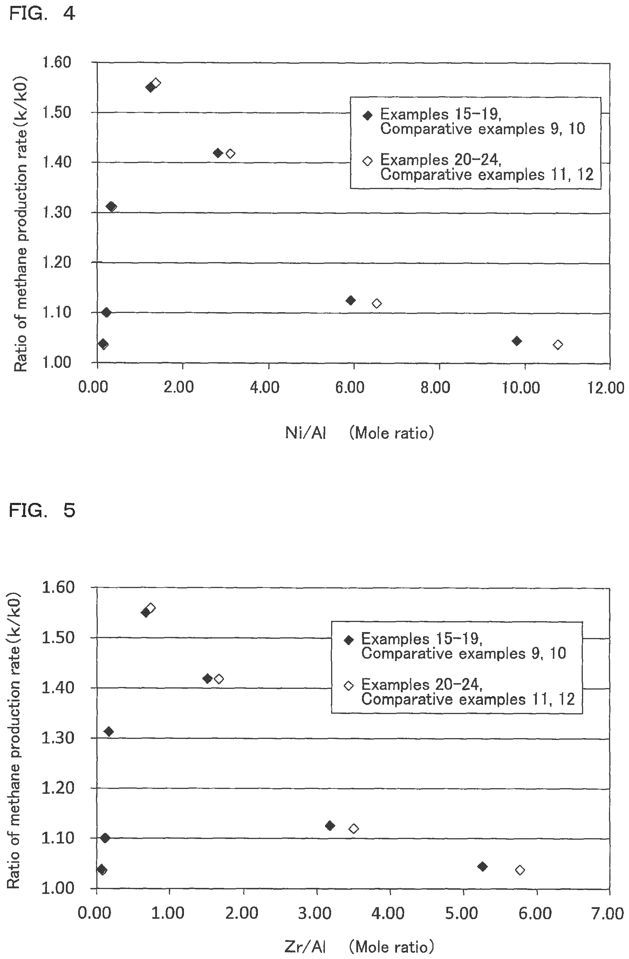

The methanation reaction catalysts (catalysts after reduction) were produced in the same manner as that of Examples 1, 3, 4, 7, and 9 and Comparative Examples 2 and 3, except that the above-described silica sol was changed to an alumina sol (trade name: alumina sol 520, manufactured by Nissan Chemical Corporation, Al₂O₃: 20 wt.%, ph = 3.0 to 5.0). The fine pore distribution and the methane production rate were measured in the same manner as those described above. The results are shown in Table 3, and FIGS. 4 and 5.

### (Examples 20 to 24 and Comparative Examples 11 and 12)

The methanation reaction catalysts (catalysts after reduction) were produced in the same manner as that of Examples 10 to 14 and Comparative Examples 7 and 8, except that the above-described alumina sol was added so that the atom ratio [Al/(Ni + Zr + Ca + Al)] of Al was the value shown in the following Table 4 instead of the above-described silica sol. The fine pore distribution and the methane production rate were measured in the same manner as those described above. The results are shown in Table 4, and FIGS. 4 and 5.

### [Table 3]

**Table 3**

| No. | Composition of Methanation Reaction Catalyst | | | | Content Ratio (wt.%) of Al₂O₃ in Catalyst before Reduction | Mole Ratio | | Fine Pore Volume | | Methane Production Rate | |
|---|---|---|---|---|---|---|---|---|---|---|---|
| | Atom Ratio (at%) | | | | | | | V[2-12nm] (cm³/g) | V/V0 | k (mmol/(s·g)) | k/k0 |
| | Ni | Zr | Sm | Al | | Ni/Al | Zr/Al | | | | |
| Comp. Ex. 9 | 58.7 | 31.5 | 3.8 | 6.0 | 3.3 | 9.81 | 5.26 | 0.046 | 1.31 | 0.067 | 1.04 |
| Ex. 15 | 56.5 | 30.3 | 3.7 | 9.5 | 5.4 | 5.93 | 3.18 | 0.050 | 1.41 | 0.072 | 1.13 |
| Ex. 16 | 51.1 | 27.4 | 3.3 | 18.2 | 10.7 | 2.81 | 1.51 | 0.057 | 1.62 | 0.091 | 1.42 |
| Ex. 17 | 41.6 | 22.3 | 2.7 | 33.4 | 21.2 | 1.25 | 0.67 | 0.100 | 2.84 | 0.099 | 1.55 |
| Ex. 18 | 20.8 | 11.2 | 1.4 | 66.7 | 51.8 | 0.31 | 0,17 | 0.185 | 5.26 | 0.084 | 1.31 |
| Ex. 19 | 15.6 | 8.4 | 1.0 | 75.0 | 61.7 | 0.21 | 0.11 | 0.188 | 5.34 | 0.070 | 1.10 |
| Comp. Ex. 10 | 11.0 | 5.9 | 0.7 | 82.4 | 71.5 | 0.13 | 0.07 | 0.188 | 5.34 | 0.066 | 1.04 |

### [Table 4]

**Table 4**

| No. | Composition of Methanation Reaction Catalyst | | | | Content Ratio (wt.%) of Al₂O₃ in Catalyst before Reduction | Mole Ratio | | Fine Pore Volume | | Methane Production Rate | |
|---|---|---|---|---|---|---|---|---|---|---|---|
| | Atom Ratio (at.%) | | | | | | | V[2-12nm] (cm³/g) | V/V0 | k (mmol/(s·g)) | k/k0 |
| | Ni | Zr | Ca | Al | | Ni/Al | Zr/Al | | | | |
| Comp. Ex. 11 | 56.8 | 30.4 | 7.6 | 5.3 | 3.1 | 10.77 | 5.76 | 0.048 | 1.30 | 0.071 | 1.04 |
| Ex. 20 | 54.9 | 29.4 | 7.3 | 8.4 | 5.0 | 6.54 | 3.50 | 0.054 | 1.48 | 0.077 | 1.12 |
| Ex. 21 | 50.2 | 26.9 | 6.7 | 16.2 | 10.0 | 3.10 | 1.66 | 0.062 | 1.70 | 0.098 | 1.42 |
| Ex. 22 | 41.8 | 22.3 | 5.6 | 30.3 | 20.0 | 1.38 | 0.74 | 0.105 | 2.87 | 0.107 | 1.56 |
| Ex. 23 | 21.9 | 11.7 | 2.9 | 63.5 | 50.0 | 0.34 | 0.18 | 0.190 | 5.20 | 0.090 | 1.31 |
| Ex. 24 | 16.6 | 8.9 | 2.2 | 72.3 | 60.0 | 0.23 | 0.12 | 0.192 | 5.23 | 0.076 | 1.10 |
| Comp. Ex. 12 | 11.8 | 6.3 | 1.6 | 80.2 | 70.0 | 0.15 | 0.08 | 0.192 | 5.24 | 0.071 | 1.04 |

Even in a case where the stabilizing element is any one of Sm and Ca, when the mole ratio (Ni/Al) of the Ni atom with respect to the Al atom is 0.20 or more and 7.0 or less, a significant increase in the total sum of the volume of the 2-12 nm fine pore was recognized, and the significant improvement of the methane production rate was confirmed.

### (Examples 25 to 29 and Comparative Examples 13 and 14)

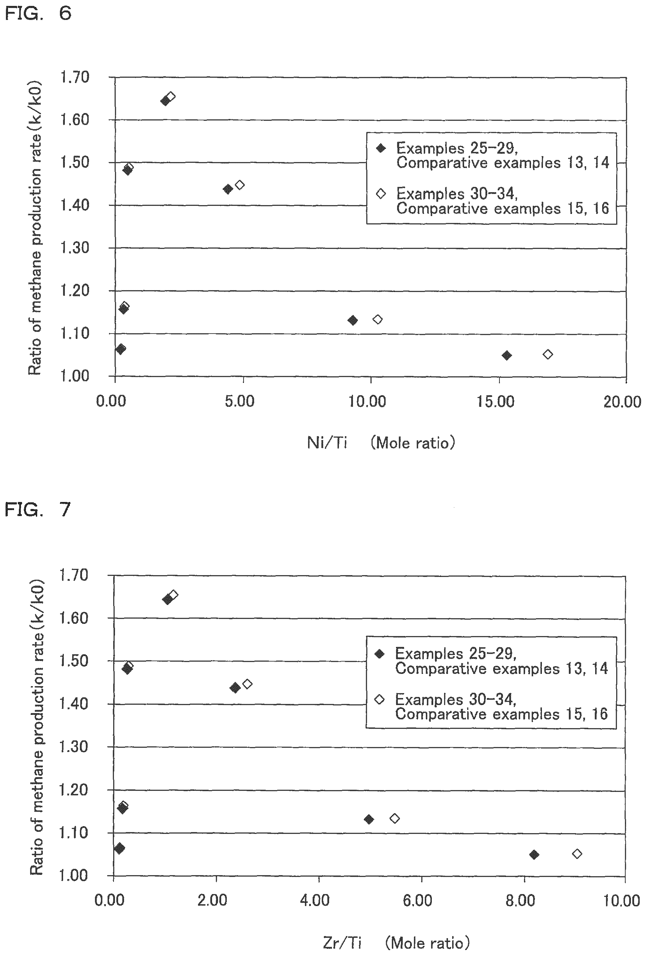

The methanation reaction catalysts (catalysts after reduction) were produced in the same manner as that of Examples 1, 3, 4, 7, and 9 and Comparative Examples 2 and 3, except that the above-described silica sol was changed to a titania sol (trade name: Tainock Am-15, manufactured by TAKI CHEMICAL CO., LTD., TiO₂: 15 wt.%, ph = 4.0). The fine pore distribution and the methane production rate were measured in the same manner as those described above. The results are shown in Table 5, and FIGS. 6 and 7.

### (Examples 30 to 34 and Comparative Examples 15 and 16)

The methanation reaction catalysts (catalysts after reduction) were produced in the same manner as that of Examples 10 to 14 and Comparative Examples 7 and 8, except that the above-described titania sol was added so that the atom ratio [Ti/(Ni + Zr + Ca + Ti)] of Ti was the value shown in the following Table 6 instead of the above-described silica sol. The fine pore distribution and the methane production rate were measured in the same manner as those described above. The results are shown in Table 6, and FIGS. 6 and 7.

### [Table 5]

**Table 5**

| No. | Composition of Methanation Reaction Catalyst | | | | Content Ratio (wt.%) of TiO₂ in Catalyst before Reduction | Mole Ratio | | Fine Pore Volume | | Methane Production Rate | |
|---|---|---|---|---|---|---|---|---|---|---|---|
| | Atom Ratio (at.%) | | | | | | | V[2-12nm] (cm³/g) | V/V0 | k (mmol/(s·g)) | k/k0 |
| | Ni | Zr | Sm | Ti | | Ni/Ti | Zr/Ti | | | | |
| Comp Ex. 13 | 60.0 | 32.2 | 3.9 | 3.9 | 3.3 | 15.28 | 8.19 | 0.045 | 1.27 | 0.067 | 1.05 |
| Ex. 25 | 58.5 | 31.4 | 3.8 | 6.3 | 5.4 | 9.28 | 4.98 | 0.055 | 1.57 | 0.072 | 1.13 |
| Ex. 26 | 54.7 | 29.3 | 3.6 | 12.4 | 10.7 | 4.40 | 2.36 | 0.065 | 1.84 | 0.092 | 1.44 |
| Ex. 27 | 7.3 | 25.4 | 3.1 | 24.2 | 21.2 | 1.95 | 1.05 | 0.121 | 3.44 | 0.105 | 1.64 |
| Ex. 28 | 27.4 | 14.7 | 1.8 | 56.1 | 51.8 | 0.49 | 0.26 | 0.197 | 5.61 | 0.095 | 1.48 |
| Ex. 29 | 21.4 | 11.5 | 1.4 | 65.7 | 61.7 | 0.33 | 0.17 | 0.199 | 5.65 | 0.074 | 1.16 |
| Comp. Ex. 14 | 15.7 | 8.4 | 1.0 | 74.9 | 71.5 | 0.21 | 0.11 | 0.200 | 5.67 | 0.068 | 1.06 |

### [Table 6]

**Table 6**

| No. | Composition of Methanation Reaction Catalyst | | | | Content Ratio (wt%) of TiO₂ in Catalyst before Reduction | Mole Ratio | | Fine Pore Volume | | Methane Production Rate | |
|---|---|---|---|---|---|---|---|---|---|---|---|
| | Atom Ratio (at.%) | | | | | | | V[2-12nm] (cm³/g) | V/V0 | k (mmol/(s·g)) | k/k0 |
| | Ni | Zr | Ca | Ti | | Ni/Ti | Zr/Ti | | | | |
| Com Ex. 15 | 57.9 | 31.0 | 7.7 | 3.4 | 3.1 | 16.88 | 9.03 | 0.048 | 1.30 | 0.072 | 1.05 |
| Ex. 30 | 56.6 | 30.3 | 7.5 | 5.5 | 5.0 | 10.25 | 5.48 | 0.059 | 1.62 | 0.078 | 1.13 |
| Ex. 31 | 53.4 | 28.5 | 7.1 | 11.0 | 10.0 | 4.85 | 2.60 | 0.070 | 1.92 | 0.100 | 1.45 |
| Ex. 32 | 46.9 | 25.1 | 6.3 | 21.7 | 20.0 | 2.16 | 1.15 | 0.127 | 3.47 | 0.114 | 1.65 |
| Ex. 33 | 28.4 | 15.2 | 3.8 | 52.6 | 50.0 | 0.54 | 0.29 | 0.207 | 5.64 | 0.102 | 1.49 |
| Ex. 34 | 22.5 | 12.0 | 3.0 | 62.5 | 60.0 | 0.36 | 0.19 | 0.208 | 5.68 | 0.080 | 1.16 |
| Comp. Ex. 16 | 16.7 | 8.9 | 2.2 | 72.2 | 70.0 | 0.23 | 0.12 | 0.208 | 5.69 | 0.073 | 1.06 |

Even in a case where the stabilizing element is any one of Sm and Ca, when the mole ratio (Ni/Ti) of the Ni atom with respect to the Ti atom is 0.30 or more and 10.5 or less, a significant increase in the total sum of the volume of the 2-12 nm fine pore was recognized, and the significant improvement of the methane production rate was confirmed.

### (Examples 35 to 39 and Comparative Examples 17 and 18)

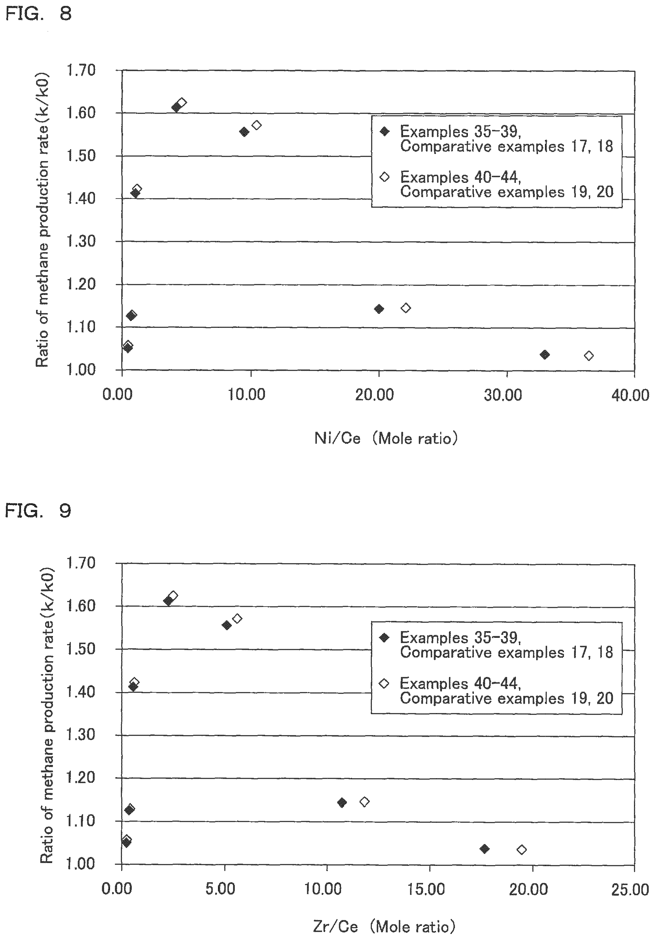

The methanation reaction catalysts (catalysts after reduction) were produced in the same manner as that of Examples 1, 3, 4, 7, and 9 and Comparative Examples 2 and 3, except that the above-described silica sol was changed to a ceria sol (trade name: CE-20A, manufactured by Nissan Chemical Corporation, CeO₂: 20 wt.%, ph = 2.0 to 4.0). The fine pore distribution and the methane production rate were measured in the same manner as those described above. The results are shown in Table 7, and FIGS. 8 and 9.

### (Examples 40 to 44 and Comparative Examples 19 and 20)

The methanation reaction catalysts (catalysts after reduction) were produced in the same manner as that of Examples 10 to 14 and Comparative Examples 7 and 8, except that the above-described ceria sol was added so that the atom ratio [Ce/(Ni + Zr + Ca + Ce)] of Ce was the value shown in the following Table 8 instead of the above-described silica sol. The fine pore distribution and the methane production rate were measured in the same manner as those described above. The results are shown in Table 8, and FIGS. 8 and 9.

### [Table 7]

**Table 7**

| No. | Composition of Methanation Reaction Catalyst | | | | Content Ratio (wt.%) of CeO₂ in Catalyst before Reduction | Mole Ratio | | Fine Pore Volume | | Methane Production Rate | |
|---|---|---|---|---|---|---|---|---|---|---|---|
| | Atom Ratio (at.%) | | | | | | | V[2-12nm] (cm³/g) | V/V0 | k (mmol/(s·g)) | k/k0 |
| | Ni | Zr | Sm | Ce | | Ni/Ce | Zr/Ce | | | | |
| Comp. Ex. 17 | 61.3 | 32.9 | 4.0 | 1.9 | 3.0 | 32.93 | 17.66 | 0.048 | 1.36 | 0.066 | 1.04 |
| Ex. 35 | 60.6 | 32.5 | 3.9 | | 5.4 | 20.00 | 10.73 | 0.051 | 1.45 | 0.073 | 1.14 |
| Ex. 36 | 58.6 | 31.4 | 3.8 | 6.2 | 10.7 | 9.48 | 5.09 | 0.069 | 1.95 | 0.100 | 1.56 |
| Ex.37 | 54.4 | 29.2 | 3.5 | 12.9 | 21.2 | 4.21 | 2.26 | 0.126 | 3.58 | 0.103 | 1.61 |
| Ex. 38 | 39.2 | 21.0 | 2.5 | 37.2 | 51.8 | 1.05 | 0.56 | 0.214 | 6.08 | 0.090 | 1.41 |
| Ex. 39 | 33.1 | 17.7 | 2.1 | 147.1 | 61.7 | 0.70 | 0.38 | 0.214 | 6.08 | 0.072 | 1.13 |
| Comp. Ex. 18 | 26.2 | 14.0 | 1.7 | 58.1 | 71.5 | 0.45 | 0.24 | 0.215 | 6.10 | 0.067 | 1.05 |

### [Table 8]

**Table 8**

| No. | Composition of Methanation Reaction Catalyst | | | | Content Ratio (wt.%) of CeO₂ in Catalyst before Reduction | Mole Ratio | | Fine Pore Volume | | Methane Production Rate | |
|---|---|---|---|---|---|---|---|---|---|---|---|
| | Atom Ratio (at.%) | | | | | | | V[2-12nm] (cm³/g) | V/V0 | k (mmol/(s·g)) | k/k0 |
| | Ni | Zr | Ca | Ce | | Ni/Ce | Zr/Ce | | | | |
| Comp. Ex. 19 | 59.0 | 31.5 | 7.9 | 1.6 | 3.1 | 36.37 | 19.46 | 0.049 | 1.34 | 0.071 | 1.04 |
| Ex. 40 | 58.4 | 31.2 | 7.8 | 2.6 | 5.0 | 22.08 | 11.82 | 0.054 | 1.47 | 0.079 | 1.15 |
| Ex. 41 | 56.7 | 30.3 | 7.6 | 5.4 | 10.0 | 10.46 | 5.60 | 0.073 | 2.00 | 0.108 | 1.57 |
| Ex. 42 | 53.1 | 28.4 | 7.1 | 11.4 | 20.0 | 4.65 | 2.49 | 0.130 | 3.56 | 0.112 | 1.62 |
| Ex. 43 | 39.5 | 21.2 | 5.3 | 34.0 | 50.0 | 1.16 | 0.62 | 0.221 | 6.03 | 0.098 | 1.42 |
| Ex. 44 | 33.8 | 18.1 | 4.5 | 43.6 | 60.0 | 0.77 | 0.41 | 0.221 | 6.05 | 0.078 | 1.13 |
| Comp. Ex. 20 | 27.2 | 14.6 | 3.6 | 54.6 | 70.0 | 0.50 | 0.27 | 0.221 | 6.05 | 0.073 | 1.06 |

Even in a case where the stabilizing element is any one of Sm and Ca, when the mole ratio (Ni/Ce) of the Ni atom with respect to the Ce atom is 0.70 or more and 25.0 or less, a significant increase in the total sum of the volume of the 2-12 nm fine pore was recognized, and the significant improvement of the methane production rate was confirmed.

### (Examples 45 to 48)

The methanation reaction catalysts (catalysts after reduction) were produced in the same manner as that of Example 4, except that the composition of the methanation reaction catalyst was changed to the atom% shown by the following Table 9.

### (Comparative Example 21 to 24)

The methanation reaction catalysts (catalysts after reduction) were produced in the same manner as that of Examples 45 to 48, except that the silica sol was not added. The fine pore distribution and the methane production rate were measured in the same manner as those described above. The results are shown in Table 9.

### (Examples 49 to 52)

The methanation reaction catalysts (catalysts after reduction) were produced in the same manner as that of Example 12, except that the composition of the methanation reaction catalyst was changed to the atom% shown by the following Table 9.

### (Comparative Example 25 to 28)

The methanation reaction catalysts (catalysts after reduction) were produced in the same manner as that of Examples 45 to 48, except that the silica sol was not added. The fine pore distribution and the methane production rate were measured in the same manner as those described above. The results are shown in Table 9.

### [Table 9]

**Table 9**

| No. | Composition of Methanation Reaction Catalyst | | | | | Content Ratio (wt.%) of SiO₂ in Catalyst before Reduction | Mole Ratio | | Fine Pore Volume | | Methane Production Rate | |
|---|---|---|---|---|---|---|---|---|---|---|---|---|
| | Atom Ratio (at.%) | | | | | | | | V[2-12nm] (cm³/g) | V/V0 | k (mmol/(s·g)) | k/k0 |
| | Ni | Zr | Sm | Ca | Si | | Ni/Si | Zr/Si | | | | |
| Comp. Ex. 21 | 25.1 | 56.4 | 18.5 | 0.0 | 0.0 | 0.0 | - | - | 0.031 | 1.00 | 0.032 | 1.00 |
| Ex. 45 | 17.5 | 39.4 | 12.9 | 0.0 | 30.1 | 20.0 | 0.58 | 1.31 | 0.124 | 3.94 | 0.057 | 1.78 |
| Comp. Ex. 22 | 29.1 | 68.6 | 2.3 | 0.0 | 0.0 | 0.0 | - | - | 0.031 | 1.00 | 0.030 | 1.00 |
| Ex. 46 | 20.3 | 48.0 | 1.6 | 0.0 | 30.1 | 20.0 | 0.68 | 1.59 | 0.120 | 3.88 | 0.053 | 1.75 |
| Comp. Ex. 23 | 50.5 | 39.5 | 10.0 | 0.0 | 0.0 | 0.0 | - | - | 0.033 | 1.00 | 0.051 | 1.00 |
| Ex. 47 | 35.3 | 27.6 | 7.0 | 0.0 | 30.1 | 20.0 | 1.17 | 0.92 | 0.135 | 4.05 | 0.092 | 1.80 |
| Comp. Ex. 24 | 78.4 | 19.8 | 1.8 | 0.0 | 0.0 | 0.0 | - | - | 0.036 | 1.00 | 0.059 | 1.00 |
| Ex. 48 | 54.8 | 13.8 | 1.3 | 0.0 | 30.1 | 20.0 | 1.82 | 0.46 | 0.149 | 4.21 | 0.105 | 1.77 |
| Comp. Ex. 25 | 29.8 | 53.8 | 0.0 | 16.5 | 0.0 | 0.0 | - | - | 0.032 | 1.00 | 0.036 | 1.00 |
| Ex. 49 | 19.8 | 35.7 | 0.0 | 11.0 | 33.6 | 20.0 | 0.59 | 1.06 | 0.129 | 3.98 | 0.063 | 1.76 |
| Comp. Ex. 26 | 45.3 | 43.8 | 0.0 | 10.9 | 0.0 | 0.0 | - | - | 0.033 | 1.00 | 0.053 | 1.00 |
| Ex. 50 | 30.1 | 29.1 | 0.0 | 7.2 | 33.6 | 20.0 | 0.90 | 0.87 | 0.126 | 3.83 | 0.096 | 1.81 |
| Comp. Ex. 27 | 74.6 | 20.4 | 0.0 | 5.0 | 0.0 | 0.0 | - | - | 0.037 | 1.00 | 0.064 | 1.00 |
| Ex. 51 | 49.5 | 13.6 | 0.0 | 3.3 | 33.6 | 20.0 | 1.48 | 0.40 | 0.152 | 4.16 | 0.114 | 1.78 |
| Comp. Ex. 28 | 74.5 | 22.1 | 0.0 | 3.4 | 0.0 | 0.0 | - | - | 0.036 | 1.00 | 0.056 | 1.00 |
| Ex.52 | 49.5 | 14.7 | 0.0 | 2.3 | 33.6 | 20.0 | 1.47 | 0.44 | 0.145 | 4.02 | 0.099 | 1.77 |

Even in a case where the composition of the methanation reaction catalyst was variously changed, when the mole ratio (Ni/Si) of the Ni atom with respect to the Si atom was 0.20 or more and 10.0 or less, a significant increase in the total sum of the volume of the 2-12 nm fine pore was recognized, and the significant improvement of the methane production rate was confirmed.

### (Example 53 and Comparative Example 29)

The methanation reaction catalysts (catalysts after reduction) were produced in the same manner as that of Example 4, except that the above-described hydrosol of the zirconia was changed to an aqueous solution of zirconium acetate oxide (trade name: Zircozol ZA-20, manufactured by DAIICHI KIGENSO KAGAKU KOGYO CO., LTD., ZrO(C₂H₃O₂)₂: 20 wt.%). In Comparative Example 29, the silica sol was not added to the mixed solution.

### (Example 54)

The methanation reaction catalyst (catalyst after reduction) was produced in the same manner as that of Example 4, except that the above-described hydrosol of the zirconia was changed to an aqueous solution of zirconium nitrate oxide (trade name: Zircozol ZN, manufactured by DAIICHI KIGENSO KAGAKU KOGYO CO., LTD., ZrO(NO₃)₂: 25 wt.%). The fine pore distribution and the methane production rate were measured in the same manner as those described above. The results are shown in Table 10.

### [Table 10]

**Table 10**

| No. | Composition of Methanation Reaction Catalyst | | | | Content Ratio (wt.%) of SiO₂ in Catalyst before Reduction | Mole Ratio | | Fine Pore Volume | | Methane Production Rate | |
|---|---|---|---|---|---|---|---|---|---|---|---|
| | Atom Ratio (at.%) | | | | | | | V[2-12nm] (cm³/g) | V/V0 | k (mmol/(s·g)) | k/k0 |
| | Ni | Zr | Sm | Si | | Ni/Si | Zr/Si | | | | |
| Comp. Ex. 29 | 62.5 | 33.5 | 4.1 | 0 | 0 | 0.00 | 0.00 | 0.037 | 1.00 | 0.023 | 1.00 |
| Ex. 53 | 43.8 | 23.5 | 2.9 | 29.8 | 21.2 | 1.47 | 0.79 | 0.140 | 3.78 | 0.116 | 5.04 |
| Ex. 54 | 43.8 | 23.5 | 2.9 | 29.8 | 21.2 | 1.47 | 0.79 | 0.141 | 3.81 | 0.115 | 5.00 |

Even in a case where the methanation reaction catalyst is produced from the salt of Zr (for example, zirconium acetate oxide, zirconium nitrate oxide, or the like), when the mole ratio (Ni/Si) of the Ni atom with respect to the Si atom is 0.20 or more and 10.0 or less, the improvement of the methane production rate was confirmed.

### (Examples 55 to 57)

The methanation reaction catalysts (catalysts after reduction) were produced in the same manner as that of Example 1, except that the above-described silica sol and the above-described alumina sol were added so that the atom ratio of Si and the atom ratio of Al shown in the following Table 11 were achieved. The fine pore distribution and the methane production rate were measured in the same manner as those described above. The results are shown in Table 11.

### [Table 11]

**Table 11**

| No. | Composition of Methanation Reaction Catalyst | | | | | Content Ratio (wt.%) of SiO₂ in Catalyst before Reduction | Content Ratio (wt.%) of Al₂O₃ in Catalyst before Reduction | Mole Ratio | | | | Fine Pore Volume | | Methane Production Rate | |
|---|---|---|---|---|---|---|---|---|---|---|---|---|---|---|---|
| | Atom Ratio (at.%) | | | | | | | | | | | V[2-12nm] (cm³/g) | V/V0 | k (mmol/(s·g)) | k/k0 |
| | Ni | Zr | Sm | Si | Al | | | Ni/Si | Zr/Si | Ni/Al | Zr/Al | | | | |
| Ex. 55 | 52.1 | 28.0 | 3.4 | 7.6 | 8.9 | 5.0 | 5.0 | 6.88 | 3.69 | 5.84 | 3.13 | 0.062 | 1.77 | 0.083 | 1.30 |
| Ex. 56 | 37.7 | 20.2 | 2.5 | 26.0 | 13.6 | 20.0 | 10.0 | 1.45 | 0.78 | 2.77 | 1.48 | 0.143 | 4.09 | 0.115 | 1.80 |
| Ex. 57 | 36.7 | 19.7 | 2.4 | 11.3 | 29.9 | 10.0 | 20.0 | 3.26 | 1.75 | 1.23 | 0.66 | 0.119 | 3.40 | 0.105 | 1.64 |

Even in a case where two or more were mixed as the inorganic oxide, a significant increase in the total sum of the volume of the 2-12 nm fine pore was recognized, and the significant improvement of the methane production rate was confirmed.

While the illustrative embodiments of the present invention are provided in the above description, such is for illustrative purpose only and it is not to be construed as limiting the scope of the present invention. Modification and variation of the present invention that will be obvious to those skilled in the art is to be covered by the following claims.

### Industrial Applicability

The methanation reaction catalyst and the method for producing methane of the present invention are preferably used in a methanation device of carbon dioxide or the like. The method for producing a methanation reaction catalyst of the present invention is preferably used in the production of the methanation reaction catalyst.

## Claims

1. A methanation reaction catalyst for subjecting CO and/or CO₂ to methanation reaction with hydrogen, and
being a calcined product of a wet mixture of zirconia and/or a salt of Zr, a salt of a stabilizing element, a salt of Ni, and an inorganic oxide, wherein
the stabilizing element is at least one of element selected from the group consisting of Y, La, Ce, Pr, Nd, Sm, Gd, Dy, Ca, Mg, Mn, Fe, and Co, and
the inorganic oxide is at least one of inorganic oxide selected from the group consisting of silica, alumina, titania, and ceria;
when the inorganic oxide contains the silica, the mole ratio of the Ni atom with respect to the Si atom is 0.20 or more and 10.0 or less;
when the inorganic oxide contains the alumina, the mole ratio of the Ni atom with respect to the Al atom is 0.20 or more and 7.0 or less;
when the inorganic oxide contains the titania, the mole ratio of the Ni atom with respect to the Ti atom is 0.30 or more and 10.5 or less; and
when the inorganic oxide contains the ceria, the mole ratio of the Ni atom with respect to the Ce atom is 0.70 or more and 25.0 or less.

2. The methanation reaction catalyst according to claim 1, wherein
when the inorganic oxide contains the silica, the mole ratio of the Zr atom with respect to the Si atom is 0.10 or more and 4.5 or less;
when the inorganic oxide contains the alumina, the mole ratio of the Zr atom with respect to the Al atom is 0.10 or more and 4.0 or less;
when the inorganic oxide contains the titania, the mole ratio of the Zr atom with respect to the Ti atom is 0.15 or more and 6.0 or less; and
when the inorganic oxide contains the ceria, the mole ratio of the Zr atom with respect to the Ce atom is 0.30 or more and 12.0 or less.

3. The methanation reaction catalyst according to claim 1, wherein
the content ratio of the inorganic oxide with respect to the total sum of the calcined product of the wet mixture of zirconia and/or the salt of Zr, the salt of the stabilizing element, the salt of Ni, and the inorganic oxide is 5 mass% or more and 62 mass% or less.

4. The methanation reaction catalyst according to claim 1 having a plurality of fine pores, wherein
the total sum of the volume of the fine pore having a fine pore diameter of 2 nm to 12 nm of the plurality of fine pores is 0.050 cm³/g or more per unit mass.

5. A method for producing methane bringing the methanation reaction catalyst according to claim 1 into contact with a mixed gas containing CO and/or Co₂ and a hydrogen gas at 200°C or more.

6. A method for producing a methanation reaction catalyst comprising the steps of:
wet-mixing zirconia and/or a salt of Zr,
at least one of inorganic oxide selected from the group consisting of silica, alumina, titania, and ceria,
a salt of at least one of stabilizing element selected from the group consisting of Y, La, Ce, Pr, Nd, Sm, Gd, Dy, Ca, Mg, Mn, Fe, and Co, and
a salt of Ni to prepare a mixture and
calcining the mixture at 400°C or more and 800°C or less, wherein
in the step of preparing the mixture,
when the inorganic oxide contains the silica, the mixture is prepared so as to have the mole ratio of the Ni atom with respect to the Si atom of 0.20 or more and 10.0 or less;
when the inorganic oxide contains the alumina, the mixture is prepared so as to have the mole ratio of the Ni atom with respect to the Al atom of 0.20 or more and 7.0 or less;
when the inorganic oxide contains the titania, the mixture is prepared so as to have the mole ratio of the Ni atom with respect to the Ti atom of 0.30 or more and 10.5 or less; and
when the inorganic oxide contains the ceria, the mixture is prepared so as to have the mole ratio of the Ni atom with respect to the Ce atom of 0.70 or more and 25.0 or less.
